(19) Europäisches Patentamt
European Patent Office
Office européen des brevets

(11) **EP 3 097 881 B1**

(12) **EUROPÄISCHE PATENTSCHRIFT**

(45) Veröffentlichungstag und Bekanntmachung des Hinweises auf die Patenterteilung:
**23.03.2022 Patentblatt 2022/12**

(21) Anmeldenummer: **16169862.6**

(22) Anmeldetag: **17.05.2016**

(51) Internationale Patentklassifikation (IPC):
*A61B 18/12* (2006.01)    *A61B 18/00* (2006.01)
*A61B 18/14* (2006.01)    *A61B 17/00* (2006.01)
*A61N 1/32* (2006.01)

(52) Gemeinsame Patentklassifikation (CPC):
**A61N 1/32; A61N 1/06; A61N 1/328;**
A61B 2017/00761; A61B 2018/00452;
A61B 2018/00464; A61B 2018/126;
A61B 2018/1495; A61B 2090/061; A61B 2090/063

(54) **VERFAHREN ZUM EINRICHTEN EINER VORRICHTUNG UND VORRICHTUNG ZUR BEHANDLUNG VON GEWEBE MITTELS ZUMINDEST EINER ZUMINDEST BIPOLAREN ELEKTRODE**

METHOD FOR CONFIGURING A DEVICE AND DEVICE FOR TREATMENT OF TISSUE USING AT LEAST ONE AT LEAST BIPOLAR ELECTRODE

PROCEDE DE CONFIGURATION D'UN DISPOSITIF ET DISPOSITIF DE TRAITEMENT DE TISSUS A L'AIDE D'AU MOINS UNE ELECTRODE AU MOINS BIPOLAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.05.2015 DE 102015108469**

(43) Veröffentlichungstag der Anmeldung:
**30.11.2016 Patentblatt 2016/48**

(73) Patentinhaber: **Wellcomet GmbH**
**76646 Bruchsal (DE)**

(72) Erfinder: **Kruglikov, Dr. Ilja**
**76351 Linkenheim-Hochstetten (DE)**

(74) Vertreter: **Lemcke, Brommer & Partner**
**Patentanwälte Partnerschaft mbB**
**Siegfried-Kühn-Straße 4**
**76135 Karlsruhe (DE)**

(56) Entgegenhaltungen:
JP-A- 2011 194 172    US-A1- 2008 183 251
US-A1- 2014 249 609

**Beschreibung**

Die Erfindung betrifft ein Verfahren zum Einrichten einer Vorrichtung zur Behandlung von Gewebe mittels zumindest einer zumindest bipolaren Elektrode gemäß Anspruch 1 und eine Vorrichtung zur Behandlung von menschlichem Gewebe mittels Hochfrequenzströmen gemäß Anspruch 4.

Es ist bekannt, eine Erwärmung von dermalen oder subkutanen Schichten menschlichen Gewebes durch Beaufschlagen des Gewebes mit hochfrequenten Strömen zu erzeugen. Hierzu wird typischerweise ein Applikator auf die Haut aufgelegt, wobei der Applikator eine monopolare, bipolare oder multipolare Elektrode umfassen kann. Mittels dieser Elektrode werden beispielsweise radiofrequente (RF) Ströme in das subkutane Gewebe eingebracht, um eine gezielte Erwärmung zu erzeugen.

Aus DE 102 24 154 A1 ist eine Vorrichtung zum elektrochirurgischen Veröden von Körpergewebe bekannt mit einer Messeinrichtung zum Messen der Impedanz des Gewebes.

Aus DE 697 33 556 T2 ist ein Verfahren zur Berührungselektrocoagulation bekannt, bei welchem ein Abstand zwischen Elektroden und Breiten der Elektroden so gewählt werden, dass eine Ablation vorbestimmter Tiefen im Gewebe erreicht wird.

Aus EP 0 873 722 A1 ist ein Gerät für eine endokardiologische Behandlung bekannt, bei dem mittels eines Transducers eine echokardiografische Dickenmessung des Myokards durchführbar ist.

Aus DE 603 12 348 T2 ist eine Elektrodenanordnung zum Versiegeln und Schneiden von Gewebe bekannt sowie das die Parameter 1) der auf das Gefäß aufgebrachte Druck und 2) der Spaltabstand zwischen leitenden Gewebekontaktflächen (Elektroden) durch die Dicke eines zu verschließenden Gefäßes beeinflusst werden.

Bei vielen Anwendungen ist es wünschenswert, eine gezielte Erwärmung von Dermis oder von subkutanem Fett durch die Beaufschlagung mit hochfrequenten Strömen zu erzielen. So ist aus US 2010/0211060 A1 ein Verfahren bekannt, bei welchem die Frequenz der hochfrequenten Ströme abhängig von der Dicke der zu beaufschlagenden Fettschicht gewählt wird. US2008/183251A1 offenbart eine Vorrichtung für nicht-invasive Hautbehandlungen durch Zuführen von RF-Energie.

Die Anwendung an menschlichem Gewebe, insbesondere zu kosmetischen, nicht therapeutischen Zwecken oder zu medizinischen Zwecken erfordert jedoch eine genaue Kenntnis der erzeugten Erwärmung und des Ortes der erzeugten Erwärmung, da andernfalls Schädigungen des Gewebes auftreten können oder der gewünschte Effekt im Zielgebiet aufgrund einer zu geringen Erwärmung und/oder einer Erwärmung an einer nicht relevanten Schicht ausbleibt.

Der vorliegenden Erfindung liegt daher die Aufgabe zugrunde, die vorbekannten Verfahren und Vorrichtungen zur Behandlung von Gewebe mittels zumindest einer zumindest bipolaren Elektrode dahingehend zu verbessern, dass eine genauere Kontrolle über die eingebrachte Energiemenge und den Ort der Erwärmung gegeben ist.

Gelöst ist diese Aufgabe durch ein Verfahren zum Einrichten einer Vorrichtung zur Behandlung von Gewebe mittels zumindest einer zumindest bipolaren Elektrode gemäß Anspruch 1. Vorteilhafte Ausgestaltungen dieses Verfahrens finden sich in den Ansprüchen 2 und 3.

Die zumindest eine Elektrode ist somit als zumindest bipolare Elektrode ausgeführt, welche somit mindestens zwei in einem Elektrodenabstand L angeordnete Elektroden aufweist.

Bei dem Verfahren zum Einrichten der Vorrichtung zur Behandlung von Gewebe mittels der zumindest einen zumindest bipolaren Elektrode wird ein Elektrodenabstand L der bipolaren Elektrode vorgegeben, wobei der Elektrodenabstand L abhängig

- von der Dicke deiner Dermisschicht des Gewebes oder hierzu korrespondierender Daten und/oder
- von einem Reflexionskoeffizienten k des Gewebes an der der Oberfläche des Gewebes abgewandten Grenze der Dermisschicht oder hierzu korrespondierender Daten

vorgegeben wird.

Die Erfindung ist auf folgender Erkenntnis des Anmelders begründet: Hochfrequente Ströme und insbesondere radiofrequente Ströme werden in einer Vielzahl unterschiedlicher Verfahren zur Behandlung von menschlichem Gewebe verwendet. Es wird davon ausgegangen, dass der wesentliche Effekt einer solchen Behandlung einer gewünschten Zielschicht des Gewebes in der Erwärmung aufgrund der eingebrachten hochfrequenten Ströme begründet ist. Zielschichten können hierbei die Dermisschicht und/oder eine tieferliegende Fettschicht (auch sWAT, subcutaneous white adipose tissue, genannt) sein.

Um eine ausreichende Temperaturerhöhung in einer Zielschicht des Gewebes (typischerweise die Dermisschicht oder die sWAT-Schicht) zu erzielen, muss eine entsprechend hohe Stromdichte in dem Zielbereich erreicht werden. Grundsätzlich wird bisher davon ausgegangen, dass durch Wahl entsprechender Parameter wie Frequenz und Amplitudenhöhe der hochfrequenten Ströme stets eine ausreichend hohe Stromdichte in dem gewünschten Zielbereich erzielt werden kann. Untersuchungen und Berechnungen des Anmelders haben jedoch gezeigt, dass die Schichtstruktur des Gewebes wesentlich die Stromflusspfade der hochfrequenten Ströme beeinflusst, so dass das Behandlungsergebnis wesentlich von den elektrischen Eigenschaften benachbarter Schichten des Gewebes, insbesondere eines elektrischen Reflexionskoeffizienten an der Grenzfläche zweier Schichten und/oder von der Dicke der einzelnen Schichten abhängt. Die Berechnungen haben ergeben, dass insbesondere die Dicke einer Dermisschicht des Gewebes und/oder der Reflexionskoeffizient des Gewebes an der der Oberfläche des Gewebes abgewandten Grenze der Dermisschicht (insbesondere an der Grenze Dermisschicht/sWAT) relevant sein können.

Diese Berechnungen des Anmelders haben gezeigt, dass die unterschiedlichen elektrischen Eigenschaften der Dermisschicht einerseits und anderer Schichten, insbesondere der sWAT-Schicht andererseits sowie der Reflexionskoeffizient zwischen diesen Schichten wesentlich das Stromflussmuster beeinflussen können und entsprechend einen signifikanten Effekt auf den Ort und die Höhe einer Temperaturerhöhung bewirken. Es stellte sich heraus, dass bisherige Annahmen über eine selektive Erwärmung einzelner Schichten, beispielsweise der sWAT-Schicht, diese vorgenannten Effekte vernachlässigen, so dass die bisher angenommenen Parameter bei einer Behandlung nicht zu der gewünschten Erwärmung führen.

Für typische Frequenzen, welche bei RF-Behandlungen angewendet werden, ist die sWAT-Schicht elektrisch von einem von außen eingeprägten Strom abgeschirmt. Hierdurch wird ein mittels einer Elektrode, beispielsweise einer bipolaren Elektrode, über die Haut eingeprägter hochfrequenter Strom in der Schichtstruktur im Wesentlichen in der Dermisschicht konzentriert, so dass sich die Erwärmung der sWAT-Schicht gegenüber bisherigen Annahmen erheblich geringer ausprägt. Im Ergebnis ist es somit wesentlich, die Konfiguration der bipolaren Elektrode abhängig von der Dicke der Dermisschicht und/oder von dem vorgenannten Reflexionskoeffizienten des Gewebes an der der Oberfläche des Gewebes abgewandten Grenze der Dermisschicht vorzugeben. Hierdurch ist eine erheblich genauere Erwärmung eines gewünschten Bereiches bzw. einer gewünschten Schicht des Gewebes, insbesondere eine wesentlich genauere Erwärmung der sWAT-Schicht möglich.

Die Berechnungen des Anmelders haben gezeigt, dass für typische Anwendungsszenarien sich der optimale Elektrodenabstand der bipolaren Elektrode für eine maximale Erwärmung eines vorgegebenen Schichtbereiches in der sWAT-Schicht um bis zu einem Faktor 2 bei korrekter Berücksichtigung der Stromflussmuster unterscheidet, von dem bisher angenommenen optimalen Elektrodenabstand ohne Berücksichtigung der durch die unterschiedlichen elektrischen Eigenschaften von Dermisschicht und sWAT-Schicht beeinflussten Stromflusspfade.

Es liegt hierbei im Rahmen der Erfindung, den Elektrodenabstand $L$ abhängig von sowohl der Dicke der Dermisschicht oder hierzu korrespondierender Daten, als auch von dem Reflexionskoeffizienten an der der Oberfläche des Gewebes abgewandten Grenze der Dermisschicht oder hierzu korrespondierender Daten vorzugeben. Hierdurch wird ein optimiertes Ergebnis erzielt.

Ebenso liegt es im Rahmen der Erfindung, einen der beiden Parameter als konstant anzunehmen und lediglich den anderen Parameter abhängig von den konkreten Gegebenheiten des zu behandelnden Gewebes zu wählen.

Untersuchungen des Anmelders haben gezeigt, dass insbesondere am menschlichen Körper der Einfluss der Dicke der Dermisschicht wesentlich sich auf die Vorgabe eines optimalen Elektrodenabstands zwischen der bipolaren Elektrode auswirkt. In einer vorteilhaften, vereinfachten Ausführungsform wird daher der Wert des Reflexionskoeffizienten als konstant, d. h. unabhängig von dem konkreten Wert des aktuell zu behandelnden Gewebes angenommen und der Elektrodenabstand der bipolaren Elektrode abhängig von der Dicke der Dermisschicht des aktuell zu behandelnden Gewebes gewählt.

Nach Auswahl des vorgegebenen Elektrodenabstandes $L$ ist die Vorrichtung eingerichtet, um eine Behandlung des Gewebes mit hochfrequenten Strömen vorzunehmen. Hierbei kann es sich um eine medizinische und/oder kosmetische, insbesondere nicht therapeutische Behandlung handeln. Insbesondere ist das Verfahren zum Einrichten einer Vorrichtung für eine nicht therapeutische Behandlung geeignet.

Eine besonders einfache Realisierung des Einrichtens der Vorrichtung zur Behandlung von Gewebe ergibt sich in einer bevorzugten Ausführungsform, wobei eine Mehrzahl von Applikatoren zur Auswahl bereitgestellt werden, wobei jeder Applikator zumindest eine zumindest bipolare Elektrode aufweist und der Elektrodenabstand der bipolaren Elektroden der genannten Applikatoren unterschiedlich ist. Hierdurch kann somit durch die Vorgabe eines speziellen Applikators die Vorgabe des Elektrodenabstandes $L$ der bipolaren Elektrode erfolgen. Insbesondere ist es vorteilhaft, dass der bestimmte Applikator durch Vorgabe eines Identifizierungszeichens, insbesondere einer Kennfarbe vorgegeben wird. In dieser bevorzugten Ausführungsform weisen die Applikatoren somit unterschiedliche Kennzeichen, wie beispielsweise Nummern, Bezeichnungen, grafische Symbole und/oder Farben auf und abhängig von der Dicke der Dermisschicht und/oder von dem vorgenannten Reflexionskoeffizienten wird somit das Identifizierungszeichen wie beispielsweise die Kennfarbe vorgegeben, so dass der Benutzer in einfacher Weise den Applikator mit dem optimalen Elektrodenabstand $L$ der bipolaren Elektrode auswählen kann. Eine weitere Vereinfachung der Handhabung wird erzielt, indem der Applikator mit dem optimalen Elektrodenabstand $L$ durch ein optisches Signal, insbesondere ein Leuchtsignal an diesem Applikator vorgegeben wird, so dass der Benutzer in einfacher Weise den Applikator mit dem aktiven optischen Signal auswählt.

Ebenso liegt es im Rahmen der Erfindung, dass ein Applikator mit einer bipolaren Elektrodenvorrichtung bereitgestellt wird, bei welchem mittels einer Steuereinheit unterschiedlicher Elektrodenabstände $L$ zwischen den Elektroden vorgebbar sind, insbesondere einstellbar sind und dass der Elektrodenabstand *L* vorgegeben wird, indem die Steuereinheit auf den Elektrodenabstand *L* konfiguriert wird. In diesem Fall erfolgt somit nach Eingabe des Parameters wie der Dicke der Dermisschicht und/oder des vorgenannten Reflexionskoeffizienten automatisch eine Konfiguration der Steuereinheit, so dass der Applikator auf den optimalen Elektrodenabstand *L* eingestellt ist.

Eine besonders einfache und fehlerunanfällige Handhabung erfolgt hierbei, indem abhängig von der Dicke *d* der Elektrodenabstand *L* vorgegeben wird, wobei eine Rechnereinheit vorgesehen ist, in welche die Dicke *d* (oder hierzu korrespondierende Daten) eingegeben wird, vorzugsweise durch einen Benutzer und die Rechnereinheit programmiert ist, den Elektrodenabstand *L* abhängig von der Dicke *d* vorzugeben.

Wesentlich ist hierbei somit, dass der Elektrodenabstand der bipolaren Elektrode abhängig von der Dicke *d* der Dermisschicht des konkret zu behandelnden Gewebes gewählt wird.

Vorteilhafterweise wird hierbei die Dicke *d* des Gewebes am Ort der Anwendung gemessen. Dies kann in an sich bekannter Weise erfolgen, insbesondere mittels Ultraschallmessung (siehe z.B. Krackowizer, P., Brenner, E. (2008) Dicke der Epidermis und Dermis, Phlebologie, 8, pp. 83-92). Besonders vorteilhaft ist hier eine weitergehende Automatisierung derart, dass eine Dickenmessungs- und Auswerteeinheit bereitgestellt wird, um aus den Messdaten der Dickenmessung die Dicke *d* zu bestimmen, wobei das Ergebnis der Dickenmessung automatisch zur Bestimmung des Elektrodenabstandes *L* verwendet wird. Der Benutzer muss in dieser vorteilhaften Ausführungsform somit die Dicke der Dermisschicht nicht manuell eingeben, sie wird automatisch aufgrund der vorgenommenen Dickenmessung ermittelt.

Die Dicke *d* der Dermisschicht variiert insbesondere abhängig vom Anwendungsort am menschlichen Körper. In einer vorteilhaften Ausführungsform wird daher die Dicke *d* abhängig von dem Anwendungsort am menschlichen Körper, insbesondere dem Anwendungsort am menschlichen Gesicht bestimmt. Hierdurch kann eine Vereinfachung erzielt werden, indem keine Dickenmessung an dem aktuell zu behandelnden Patienten vorgenommen werden muss. Vielmehr kann auf Durchschnittswerte der Dicke der Dermisschicht an dem aktuellen Behandlungsort, wie beispielsweise der Stirn, der Wange oder eines anderen Bereichs des menschlichen Körpers zurückgegriffen werden. Diese Durchschnittswerte können beispielsweise tabellarisch erfasst und entsprechend durch den Benutzer ausgewählt werden. Besonders vorteilhaft ist es, dass der Benutzer den Behandlungsort in eine Rechnereinheit eingibt, welche derart programmiert ist, dass sie abhängig von dem Behandlungsort den optimalen Elektrodenabstand L zwischen den bipolaren Elektroden ermittelt. Der Behandlungsort am menschlichen Körper stellt in diesem Fall somit ein zu der Dicke der Dermisschicht korrespondierendes Datum dar.

Ein Verfahren zur Behandlung von menschlichem Gewebe, insbesondere zur nicht therapeutischen Behandlung von menschlichem Gewebe, mit zumindest einer zumindest bipolaren Elektrode umfasst die Verfahrensschritte, dass in einem Verfahrensschritt a eine Vorgabe eines Elektrodenabstands L zwischen den Elektroden der bipolaren Elektrode erfolgt, mittels des vorangehend beschriebenen Verfahrens zum Einrichten einer Vorrichtung zur Behandlung von Gewebe mittels zumindest einer zumindest bipolaren Elektrode, insbesondere einer bevorzugten Ausführungsform hiervon. In einem Verfahrensschritt b erfolgt dann das Behandeln des menschlichen Gewebes, insbesondere das nicht therapeutische Behandeln des menschlichen Gewebes durch Beaufschlagen mit hochfrequenten Strömen mittels der bipolaren Elektrode.

Die der Erfindung zugrunde liegende Aufgabe ist weiterhin durch eine Vorrichtung zur Behandlung von menschlichem Gewebe mittels Hochfrequenzströmen gemäß Anspruch 4 gelöst. Vorteilhafte Ausgestaltungen der erfindungsgemäßen Vorrichtung finden sich in den Ansprüchen 5 und 6.

Die erfindungsgemäße Vorrichtung weist einen Generator zum Erzeugen von Hochfrequenzströmen auf, zumindest eine erste und zumindest eine zweite zumindest bipolare Elektrode. Die bipolaren Elektroden sind mit einem Generator elektrisch leitend verbunden, wobei die beiden bipolaren Elektroden einen unterschiedlichen Elektrodenabstand L aufweisen. Wesentlich ist, dass ein Vorgabemittel zur Vorgabe des Elektrodenabstands L abhängig

- von der Dicke *d* einer Dermisschicht des Gewebes oder hierzu korrespondierender Daten und/oder
- von einem Reflexionskoeffizienten *k* des Gewebes an der der Oberfläche des Gewebes abgewandten Grenze der Dermisschicht oder hierzu korrespondierender Daten

vorgesehen ist.

Die erfindungsgemäße Vorrichtung ermöglicht es somit eine erheblich präzisere Behandlung, da erstmals die eingangs erwähnte Erkenntnis Einfluss in die Ausgestaltung einer solchen Vorrichtung findet, dass für eine präzise Vorgabe des Ortes der gewünschten Erwärmung die Wahl des Elektrodenabstandes L zwischen den Elektroden abhängig von einem oder beiden der vorgenannten Parameter wesentlich ist. Hierdurch ergeben sich die eingangs erwähnten Vorteile.

In einer vorteilhaften Ausgestaltung weist die Vorrichtung einen Applikator auf, welcher elektrisch leitend mit dem Generator verbunden ist. Die Vorrichtung weist weiterhin zumindest eine erste Wechselkappe mit der ersten bipolaren Elektrode und eine zweite Wechselkappe mit der zweiten bipolaren Elektrode auf, welche Wechselkappen wahlweise an dem Applikator anordbar ausgebildet sind, derart, dass die bipolare Elektrode der an dem Randstück angeordneten Wechselkappe elektrisch leitend mit dem Generator verbunden ist.

In dieser bevorzugten Ausführungsform kann die Auswahl des optimalen Elektrodenabstands *L* somit durch Auswahl der entsprechenden Wechselkappe erfolgen. Insbesondere ist es hierbei vorteilhaft, die Wechselkappen mit Identifizierungszeichen, beispielsweise Nummern, Symbolen und/oder farblichen Markierungen zu versehen. Wie bereits bei den Verfahren zum Einrichten einer Vorrichtung zur Behandlung von Gewebe ausgeführt ist es hierbei vorteilhaft, dass die Vorrichtung eine Kennungsanzeigeeinheit umfasst, welche ausgebildet ist, abhängig von dem vorgegebenen Elektrodenabstand *L* die Kennung der entsprechenden Wechselkappe auszugeben.

Es liegt im Rahmen der Erfindung eine Mehrzahl von Wechselkappen mit unterschiedlichen Elektrodenabständen vorzusehen, insbesondere im Bereich 5 bis 20 Wechselkappen.

In einer weiteren bevorzugten Ausführungsform weist die Vorrichtung zumindest einen ersten und einen zweiten Applikator auf, wobei die erste Elektrode an dem ersten Applikator und die zweite Elektrode an dem zweiten Applikator angeordnet ist und wobei jeder Applikator eine optische Anzeige, insbesondere ein Leuchtmittel aufweist. Das Vorgabemittel ist elektrisch mit der optischen Anzeige des Applikators verbunden und derart ausgebildet, dass abhängig von dem vorgegebenen Elektrodenabstand *L* die optische Anzeige des entsprechenden Applikators aktiviert wird. Hierdurch wird eine besonders einfache und fehlerunanfällige Benutzung ermöglicht.

Das Vorgabemittel weist eine Eingabeeinheit zur Eingabe

- von der Dicke *d* einer Dermisschicht des Gewebes oder hierzu korrespondierender Daten und/oder
- von einem Reflexionskoeffizienten *k* des Gewebes an der der Oberfläche des Gewebes abgewandten Grenze der Dermisschicht oder hierzu korrespondierender Daten

auf. Das Vorgabemittel weist weiterhin eine Auswerteeinheit zur Bestimmung des vorzugebenden Elektrodenabstandes *L* auf. Eingabeeinheit und Auswerteeinheit können insbesondere als handelsüblicher Rechner mit entsprechenden Eingabe und Anzeigevorrichtungen wie Bildschirm, Tastatur und/oder berührungsempfindlichen Bildschirmen ausgebildet seien.

Die Eingabeeinheit ist bevorzugt zur Eingabe zu der Dicke *d* korrespondierender Daten ausgebildet, indem durch ein Benutzer ein Anwendungsort am menschlichen Körper auswählbar ist, insbesondere aus einer durch die Eingabeeinheit vorgegebenen Liste von Anwendungsarten. In dieser bevorzugten Ausführungsform ist die Bedienung somit erheblich vereinfacht, indem der Benutzer lediglich den Anwendungsort eingibt und abhängig von dieser Eingabe, welche Rückschlüsse auf die Dicke *d* der Dermisschicht an dem gewünschten Anwendungsort zulässt, der Elektrodenabstand *L* der bipolaren Elektrode vorgegeben wird. Eine besonders einfache Eingabemöglichkeit ergibt sich hierbei, indem der menschliche Körper auf einem berührungsempfindlichen Bildschirm dargestellt ist und der Benutzer durch Antippen der entsprechenden Stelle den gewünschten Behandlungsort auswählt und entsprechend der für eine typische Dicke der Dermisschicht an diesen Behandlungsort optimale Elektrodenabstand *L* vorgegeben wird.

Eine weitere Erhöhung der Genauigkeit wird erzielt, indem die Vorrichtung eine Dickenmesseinheit zur Dickenmessung der Dicke *d* der Dermisschicht umfasst, z.B. eine Ultraschalldickenmesseinheit und die Dickenmesseinheit mit dem Vorgabemittel zusammenwirkend ausgebildet ist, um eine gemessene Dicke *d* an das Vorgabemittel weiterzuleiten, insbesondere automatisch weiterzuleiten. Bei Benutzung wird somit an dem konkreten Behandlungsort des zu behandelnden Gewebes die Dicke bestimmt, bevorzugt mittels Ultraschallmessung und anschließend abhängig von der gemessenen Dicke der Dermisschicht ein optimaler Elektrodenabstand *L* der bipolaren Elektrode vorgegeben.

Die erfindungsgemäße Vorrichtung ist bevorzugt zur Durchführung des erfindungsgemäßen Verfahrens, insbesondere einer bevorzugten Ausführungsform hiervon ausgebildet. Das erfindungsgemäße Verfahren ist bevorzugt zur Durchführung mittels der erfindungsgemäßen Vorrichtung, insbesondere einer bevorzugten Ausführungsform hiervon, ausgebildet.

Je nach Art der Anwendung können für die Erwärmung der Zielschicht unterschiedliche Optimierungsmodelle von Interesse sein: So kann es in einem ersten, Punktoptimierungsmodell von Interesse sein, den optimalen Elektrodenabstand *L* für die Erwärmung in einer vorgegebenen Tiefe z zu ermitteln. Für andere Anwendungen kann es von Interesse sein, in einem Bereichsoptimierungsmodell den optimalen Elektrodenabstand *L* für eine Erwärmung einer Tiefenschicht, d. h. eines Tiefenbereichs beginnend mit einer Tiefe $h_1$ bis zu einer Tiefe $h_2$ zu erwärmen.

Im Folgenden wird als bevorzugte Ausführungsform eine Formel und numerische Beispiele zur Ermittlung des optimalen Elektrodenabstands L für eine Erwärmung in einer Tiefe z der subkutanen Fettschicht sWAT dargestellt. Die Tiefe z stellt somit den Abstand senkrecht zur Hautoberfläche und gemessen ausgehend von der Hautoberfläche (der Hautgrenze nach außen) dar.

Analysen und Berechnungen des Erfinders haben ergeben, dass in diesem Fall der optimale Abstand *L* durch beispielsweise numerisches Lösen der folgenden Formel 1 erhalten werden kann:

$$\sum_{n=0}^{\infty} k^n \frac{4\left[2n+\mu\right]^2 - 2v^2}{\left\{v^2 + 4\left[2n+\mu\right]^2\right\}^{5/2}} = 0$$

(Formel 1)

Hierbei bezeichnet **k** den Reflexionskoeffizienten an der Grenzfläche Dermis/sWAT. Dieser Reflexionskoeffizient ist gemäß folgender Formel 2 definiert:

$$k = \frac{\sigma_d - \sigma_s}{\sigma_d + \sigma_s}$$

(Formel 2)

mit den elektrischen Konduktivitäten $\sigma_d$ der Dermisschicht und $\sigma_s$ der sWAT-Schicht. Hieran ist bereits ersichtlich, dass der optimale Abstand nicht von den Absolutwerten der elektrischen Konduktivitäten dieser Schichten abhängt, sondern lediglich von deren Verhältnis gemäß Definition des Reflexionskoeffizienten **k**.

$\mu$ gibt das Verhältnis der gewünschten Tiefe z zu der Dicke **d** der Dermisschicht gemäß Formel 3 wieder:

$$z = \mu d$$

(Formel 3)

v gibt das Verhältnis des optimalen Abstandes **L** der Elektroden zu der Dicke der Dermisschicht **d** gemäß Formel 4 wieder:

$$L_1^{lay} = v d$$

(Formel 4)

Zur Verdeutlichung des hier zugrunde liegenden Modells wird L auch als $L_1^{lay}$ bezeichnet.

Wird nun beispielsweise durch den Benutzer die Dicke **d** der Dermisschicht sowie der Reflexionskoeffizient **k** zwischen Dermis- und sWAT-Schicht vorgegeben, so kann durch an sich bekannte numerische Verfahren gemäß obiger Formel 1 für eine von dem Benutzer vorgegebene Tiefe z in der sWAT-Schicht der optimale Elektrodenabstand L für die Anwendung ermittelt und entsprechend bei der Anwendung berücksichtigt werden.

Grundsätzlich liegt es im Rahmen der Erfindung, dass eine Mehrzahl diskreter Elektrodenabstände vorgesehen sind, beispielsweise aufgrund des Vorsehens einer Mehrzahl von Wechselkappen mit unterschiedlichen Elektrodenabständen wie zu oben beschrieben oder beispielsweise von mehreren Applikatoren mit unterschiedlichen Elektrodenabständen. In diesen Fällen erfolgt die Optimierung vorzugsweise derart, dass der dem berechneten optimalen Abstand nächstliegende vorhandene Elektrodenabstand für die Anwendung verwendet wird bzw. dem Benutzer entsprechend signalisiert wird, beispielsweise durch Vorgabe der entsprechenden Wechselkappe oder Kennzeichnung des entsprechenden Applikators.

Für eine bestmögliche Optimierung des Elektrodenabstands ist es vorteilhaft, wenn sowohl der Reflexionskoeffizient **k,** als auch die Dicke **d** der Dermisschicht abhängig von dem jeweiligen Anwendungsort vorgegeben werden und abhängig von diesen beiden Parametern wie zuvor beschrieben der optimale Elektrodenabstand ermittelt wird. Untersuchungen des Erfinders haben jedoch ergeben, dass die Abhängigkeit von der Dicke der Dermisschicht zu einer größeren Variation des optimalen Elektrodenabstands **L** führt, verglichen mit der Variation des Reflexionskoeffizienten für typische Anwendungsorte am menschlichen Körper. In einer vorteilhaften Ausgestaltung wird daher der Reflexionskoeffizient als konstant, d. h. unabhängig vom Anwendungsort angenommen und lediglich die Dicke der Dermisschicht abhängig von dem Anwendungsort gewählt. In diesem Fall kann der Reflexionskoeffizient **k** bevorzugt mit 0,905 angenommen werden.

Die nachfolgende Tabelle 1 zeigt beispielhafte Ergebnisse, welche aus der numerischen Auswertung der Formel 1 resultieren:

Tabelle 1

| $\mu = z/d$ | $v = L_1^{lay}/d$ | $L_1^{lay}/L_1^{hom}$ |
|---|---|---|
| 1 | 1.487 | 1.051 |
| 2 | 3.272 | 1.157 |
| 3 | 5.250 | 1.237 |
| 4 | 7.275 | 1.286 |

In der ersten Spalte ist das Verhältnis $\mu$ angegeben, welches das Verhältnis zwischen der gewünschten Tiefe z und der Dicke d der Dermisschicht wiedergibt. Ein Wert $\mu=1$ bedeutet somit, dass eine Optimierung für eine Tiefe in der sWAT-Schicht gewünscht ist, welche der Grenze Dermisschicht/sWAT-Schicht entspricht. In Spalte 2 ist das Verhältnis v des optimalen Elektrodenabstands L zu der Dicke der Dermisschicht angegeben. Der Wert 1,487 für $\mu$ = 1 bedeutet somit, dass in diesem Fall die Elektroden im optimalen Fall einen Abstand aufweisen, welcher dem 1,487-fachen der Dicke der Dermisschicht entspricht. In der dritten Spalte ist zur Verdeutlichung der Relevanz der vorliegenden Erfindung ein Optimierungswert angegeben, welcher sich ergeben würde, wenn man die Inhomogenität der elektrischen Konduktivitäten in Dermis und sWAT-Schicht vernachlässigen würde und somit das gesamte Gewebe als hinsichtlich der elektrischen Konduktivität homogen annehmen würde. Angegeben ist das Verhältnis des gemäß der vorliegenden Erfindung ermittelten optimalen Abstandes L zu dem Abstand $L_1^{hom}$, der sich bei Annahme des elektrisch homogenen Gewebes ergeben würde.

Tabelle 1 zeigt, dass beispielsweise bei einer gewünschten Optimierung für eine Tiefe, welche dem vierfachen der Dermisschicht entspricht (letzte Zeile der Tabelle) die Abweichung des optimalen Elektrodenabstands gemäß der vorliegenden Erfindung gegenüber den bisherigen angenommenen Werten über 28 % beträgt!

Als numerisches Beispiel kann eine typische Dicke der Dermisschicht von 0,5 mm angenommen werden. Wird nun eine Behandlung in der sWAT-Schicht in einer Tiefe von 1 mm gewünscht, d. h. eine Optimierung des Elektrodenabstandes derart, dass ein maximaler Energieeintrag in einem Abstand von 0,5 mm in der sWAT-Schicht zu der Grenzschicht Dermis/sWAT-Schicht erfolgt gewünscht, so ergibt sich der optimale Elektrodenabstand aus Spalte 2 der Tabelle 1: $\mu$ beträgt in diesem Fall 2 und somit beträgt das Verhältnis von L zur Dicke *d* der Dermisschicht 3,272 gemäß Tabelle 1. Hieraus ergibt sich mit einer Dicke 0,5 mm der Dermisschicht für diese gewünschte Anwendung somit ein optimaler Elektrodenabstand L von 1,636 mm.

Wie zuvor ausgeführt, gibt Tabelle 1 Berechnungsbeispiele für einen als konstant, d. h. ortsunabhängig angenommenen Reflexionskoeffizienten *k* = 0,905 wieder.

In der nachfolgenden Tabelle 2 ist die Abhängigkeit von dem Reflexionskoeffizienten verdeutlicht:

Tabelle 2

| *k* | $v = L_1^{lay}/d$ | $L_1^{lay}/L_1^{hom}$ |
|---|---|---|
| 0.950 | 3.323 | 1.175 |
| 0.905 | 3.272 | 1.157 |
| 0.800 | 3.178 | 1.124 |
| 0.700 | 3.109 | 1.099 |

Tabelle 2 gibt den optimalen Elektrodenabstand für eine gewünschte Behandlung in einer Tiefe welche dem doppelten der Dicke der Dermisschicht entspricht für unterschiedliche Reflexionskoeffizienten *k* wieder. In der ersten Spalte ist entsprechend der Reflexionskoeffizient *k* angegeben. Die zweite Spalte zeigt wie auch bei Tabelle 1 das Verhältnis des optimalen Elektrodenabstands *L* zu der Dicke *d* der Dermisschicht. Spalte 3 zeigt ebenfalls wie auch bei Tabelle 1 das Verhältnis des gemäß der vorliegenden Erfindung und Formel 1 ermittelten optimalen Elektrodenabstands *L* zu einem ermittelten Elektrodenabstand $L^{hom}$ bei Annahme des Gewebes mit homogener elektrischer Konduktivität wieder.

Die Spalte 2 gemäß Tabelle 2 mit *k* = 0,905 entspricht somit den Vorgaben der Berechnungen zu Tabelle 1 mit $\mu$ = 2.

Als weiteres Ausführungsbeispiel wird nachfolgend eine Berechnungsformel angegeben, wenn eine Optimierung des Elektrodenabstands für einen Tiefenbereich in der sWAT-Schicht erfolgen soll, wobei der Tiefenbereich beginnend mit einer ersten Tiefe $h_1$ und endend mit einer zweiten Tiefe $h_2$ durch den Benutzer vorgegeben wird. Analog zu der Tiefe z stellen die Tiefen $h_{1,2}$ den Abstand senkrecht zur Hautoberfläche in der sWAT-Schicht von der Hautoberfläche dar.

Der optimale Elektrodenabstand $L$ ergibt sich hierbei durch nachfolgende Formel 5

$$\sum_{n=0}^{\infty} k^n \left\{ \frac{2n + \mu_1}{v^2 + 4\left(2n + \mu_1\right)^2} - \frac{2n + \mu_2}{v^2 + 4\left(2n + \mu_2\right)^2} \right\} = 0$$

(Formel 5)

Wie auch bei Formel 1 bezeichnet $k$ hier den Reflexionskoeffizienten gemäß Formel 2. $\mu_1$ bezeichnet analog Formel 3 das Verhältnis zwischen $h_1$ und der Dicke der Dermisschicht und entsprechend $\mu_2$ das Verhältnis zwischen $h_2$ und der Dicke der Dermisschicht:

$$h_{1,2} = \mu_{1,2}d$$

(Formel 6)

Zur Verdeutlichung des hier zugrunde liegenden Modells wird $L$ auch als $L_2^{lay}$ bezeichnet. $L_2^{lay}$ ergibt sich beispielsweise aus der numerischen Lösung der Formel 5, wobei auch hier v das Verhältnis des optimalen Abstandes $L$ der Elektroden zu der Dicke der Dermisschicht $d$ angibt:

$$L_2^{lay} = vd$$

(Formel 7)

In Tabelle 3 ist für unterschiedliche Reflexionskoeffizienten $k$ und unterschiedliche vorgegebene Schichten $[h_1, h_2]$ ("Strip") wiederrum in Spalte 3 das Verhältnis v des sich ergebenden optimalen Elektrodenabstands $L_2^{lay}$ zu der Dicke der Dermisschicht $d$ angegeben und in Spalte 4 das Verhältnis des optimalen Elektrodenabstandes zu einem ermittelten Elektrodenabstand $L_2^{hom}$ bei Annahme des Gewebes mit einer homogenen elektrischen Konduktivität.

Tabelle 3

| $k$ | Strip | $L_2^{lay} / d$ | $L_2^{lay} / L_2^{hom}$ |
|---|---|---|---|
| 0.800 | [$d$,2$d$] | 4.205 | 1.487 |
| 0.905 | [$d$,2$d$] | 5.203 | 1.840 |
| 0.950 | [$d$,2$d$] | 6.386 | 2.258 |
| 0.800 | [2$d$,3$d$] | 7.706 | 1.573 |
| 0.905 | [2$d$,3$d$] | 9.781 | 1.997 |
| 0.950 | [2$d$,3$d$] | 12.182 | 2.487 |

Wird beispielsweise eine Optimierung für eine Teilschicht der sWAT-Schicht gewünscht, beginnend mit einer Tiefe entsprechend der Dicke der Dermisschicht $d$ und endend mit einer Tiefe entsprechend der doppelten Dicke der Dermisschicht $2d$, so ergibt sich für einen typischen Reflexionskoeffizienten $k$ von 0,905 gemäß Zeile 2 der Tabelle 3 somit ein Verhältnis von 5,203 des optimalen Elektrodenabstandes zu der Dermisschicht. Wird somit beispielsweise bei einer Dicke von 0,5 mm der Dermisschicht eine optimale Erwärmung in einer Tiefe 0,5 mm bis 1 mm in der sWAT-Schicht gewünscht, so beträgt gemäß Zeile 2 das Verhältnis des optimalen Elektrodenabstands $L$ zu der Dicke der Dermisschicht 5,203 und somit in diesem Fall der optimale Elektrodenabstand 2,601 mm. Gemäß Spalte 4, Zeile 2 der Tabelle 3 weicht dieses Ergebnis um über 125 % von dem Ergebnis bei Annahme einer homogenen elektrischen Konduktivität des Gewebes ab!

Entsprechend würde bei den vorgenannten Parametern, jedoch einer gewünschten optimalen Erwärmung in 1 mm bis 1,5 mm der sWAT-Schicht gemäß Zeile 5 der Tabelle 3 der optimale Elektrodenabstand das 9,781-fache der Dicke der Dermisschicht betragen, d. h. etwa 4,890 mm.

Typische optimale Abstände *L* zwischen den Elektroden (d.h. den Einzelelektroden) einer bipolaren Elektrode liegen im Bereich 0,5 mm bis 10 mm, insbesondere 1 mm bis 5 mm. Vorzugsweise liegen daher die Abstände L der bipolaren Elektroden der erfindungsgemäßen Vorrichtung, insbesondere einer bevorzugten Ausführungsform hiervon, in diesem Bereich.

Die zumindest bipolaren Elektroden weisen wie zuvor beschrieben somit mindestens zwei in einem Elektrodenabstand *L* angeordnete Elektroden (auch Einzelelektroden genannt) auf.

Die Kontaktfläche jeder Elektrode zum Einprägen von Strom in das Gewebe kann hierbei unterschiedliche Formen aufweisen: Vorteilhaft ist insbesondere eine kreisförmige Kontaktfläche, welche technisch unaufwändig herstellbar ist. Ebenso kann die Kontaktfläche als linenartig, sich länglich erstreckend ausgebildet sein, wobei hierbei die beiden Elektroden bevorzugt parallel verlaufend nebeneinander angeordnet sind. Kreisförmige Kontaktflächen weisen bevorzugt einen Durchmesser im Bereich 0,5 mm bis 10 mm, insbesondere im Bereich 1 mm bis 3 mm auf.

Weiterhin kann eine Elektrode mehrere Kontaktflächen umfassen. Insbesondere ist es vorteilhaft, eine Elektrode mit mehreren bevorzugt kreisförmigen Kontaktflächen auszubilden, welche bevorzugt linenartig, nach Art einer gepunkteten Linie angeordnet sind. Auch hier sind bevorzugt die beiden nach Art einer gepunkteten Linie ausgebildeten Elektroden parallel angeordnet. Die einzelnen Kontakflächen einer Elektrode könnten hierbei einen Abstand (zwischen den Flächenmittelpunkten) im Bereich 0,5 mm bis 10 mm, bevorzugt 1 mm bis 5 mm aufweisen.

Der Elektrodenabstand *L* bezeichnet vorzugsweise und insbesondere bei den vorangengenen Berechnungen den Abstand zwischen den Flächenmittelpunkten der beiden Elektroden.

Auf einem Applikator können mehrere bipolare Elektroden nebeneinander angeordnet sein, so dass ein größerer Flächenbereich gleichzeitig mit Strömen beaufschlagt werden kann.

Die Parameter der Ströme, welche mittels der Elektroden in das Gewebe eingebracht werden, können an sich bekannten Paramteren, insbesondere bekannten Paramtern bei der RF-Behandlung entsprechen. Bevorzugt liegen die Frequenzen der RF-Ströme im Bereich 0,5 MHz bis 10 MHz, insbesondere 1 MHz bis 5 MHz.

Weitere bevorzugte Merkmale und Ausführungsformen werden im Folgenden anhand von Ausführungsbeispielen und den Figuren erläutert. Dabei zeigt:

Figur 1    ein erstes Ausführungsbeispiel einer erfindungsgemäßen Vorrichtung mit Wechselkappen für einen Applikator;

Figur 2    ein zweites Ausführungsbeispiel einer erfindungsgemäßen Vorrichtung mit zwei Applikatoren,

Figur 3    ein drittes Ausführungsbeispiel einer erfindungsgemäßen Vorrichtung mit einer Einheit zur Dickenmessung der Dermisschicht und

Figur 4    ein weiteres Ausführungsbeispiel eines Applikators mit mehreren bipolaren Elektroden.

Die Figuren zeigen schematische, nicht maßstabsgetreue Darstellungen. Gleiche Bezugszeichen in den Figuren bezeichnen gleiche oder gleichwirkende Elemente.

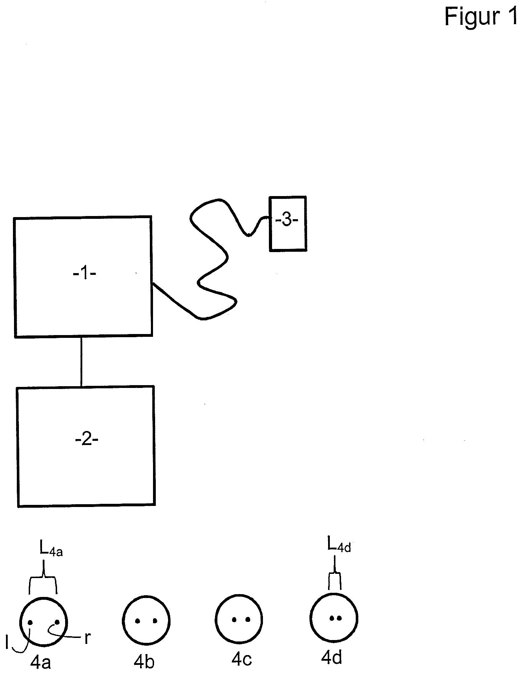

Das in Figur 1 dargestellte erste Ausführungsbeispiel einer Vorrichtung zur Behandlung von menschlichem Gewebe mittels Hochfrequenzströmen weist einen Generator 1 zum Erzeugen von Hochfrequenzströmen auf. Der Generator 1 ist über ein flexibles Kabel mit einem Applikator 3 verbunden. Der Applikator 3 weist eine Wechselkappe mit einer bipolaren Elektrode auf, wobei in Figur 1 unten beispielhaft vier Wechselkappen 4a bis 4d abgebildet sind, von denen wahlweise eine an dem Applikator 3 angeordnet werden kann.

Die Vorrichtung gemäß Figur 1 weist weiterhin ein Vorgabemittel 2 zur Vorgabe eines Elektrodenabstands *L* abhängig von einer Dicke *d* einer Dermisschicht des Gewebes auf, welches mittels Hochfrequenzströmen beaufschlagt werden soll.

Das Vorgabemittel 2 weist weiterhin Eingabemittel auf, mittels derer der Benutzer weitere Parameter der Behandlung, wie beispielsweise Frequenz und Amplitude der Hochfrequenzströme vorgeben kann. Die Eingabemittel sind vorliegend als berührungsempfindlicher Bildschirm ausgebildet, ebenso sind andere Ausbildungen wie beispielsweise als Tastatur möglich.

Die Wechselkappen weisen jeweils mindestens eine bipolare Elektrode auf, mit einer linken Elektrode und einer rechten Elektrode. Beispielhaft sind für die Wechselkappe 4a die linke Elektrode *l* und die rechte Elektrode r gekennzeichnet. Die bipolare Elektrode weist einen Elektrodenabstand *L* auf. Dieser ist beispielhaft für die Wechselkappe 4a mit $L_{4a}$ und für die Wechselkappe 4d mit $L_{4d}$ gekennzeichnet. Die Wechselkappen 4a bis 4d weisen somit paarweise verschiedene Elektrodenabstände *L* auf.

Der Benutzer gibt nun in das Vorgabemittel 2 eine Dicke *d* der Dermisschicht des Gewebes an dem zu behandelnden Ort ein. Ebenso gibt er eine Tiefe z ein, in welcher in einer sWAT-Schicht unterhalb der Dermisschicht eine Erwärmung mittels Energieeintrag erfolgen soll. Das Vorgabemittel 2 umfasst eine Rechnereinheit, welche durch numerische Auswertung der Formel 1 abhängig von der vorgegebenen Dicke *d* und der gewünschten Tiefe *z* einen optimalen Elektrodenabstand *L* berechnet. Hierzu ist ein Reflexionskoeffizient *k* = 0,905 fest vorgegeben. Dieser Reflexionskoeffizient kann jedoch bei Bedarf von dem Benutzer abgeändert werden.

Weiterhin sind in der Rechnereinheit des Vorgabemittels 2 die Elektrodenabstände *L* der bipolaren Elektroden der Wechselkappen 4a bis 4d vorgegeben. Die Rechnereinheit ermittelt diejenige Elektrodenkappe, welche den nächstliegenden Elektrodenabstand *L* zu dem ermittelten optimalen Elektrodenabstand aufweist und gibt dem Benutzer eine entsprechende Kennung dieser Elektrodenkappe aus. Vorliegend sind die Elektrodenkappen mit Buchstaben a, b, c und d gekennzeichnet und entsprechend wird am Vorgabemittel der Buchstabe derjenigen Wechselkappe angezeigt, welche dem optimalen Elektrodenabstand am nächsten kommt. Alternativ sind andere Kennungen, insbesondere farbliche Kennungen zur Auswahl der Wechselkappen möglich.

Der Benutzer nimmt nun die durch das Vorgabemittel angezeigte Wechselkappe und steckt sie auf den Applikator 3. Applikator 3 und Wechselkappe sind derart ausgebildet, dass bei Aufstecken durch den Benutzer eine elektrisch leitende Verbindung der bipolaren Elektrode der Wechselkappe mit dem Applikator 3 und somit über das flexible Kabel mit dem Generator 1 besteht.

Anschließend legt der Benutzer mittels des Applikators 3 die bipolare Elektrode der auf diesen Applikator aufgesteckten Wechselkappe auf das Gewebe auf zur Beaufschlagung des Gewebes mit Hochfrequenzströmen, welche durch den Generator 1 erzeugt werden.

Die Wechselkappen 4a bis 4d weisen vorliegend folgende Abstände L und folgende Durchmesser der Kontaktflächen der Elektroden der jeweiligen bipolaren Elektrode auf:

Tabelle 4

| Wechsel kappe | Elektrodenabstand *L* [mm] | Elektrodendurchmesser [mm] |
|---|---|---|
| 4a | 4,5 | 1,5 (1 bis 2) |
| 4b | 3 | 1,0 (0,8 bis 1,2) |
| 4c | 1,5 | 0,4 (0,3 bis 0,6) |
| 4d | 1 | 0,3 (0,2 bis 0,4) |

Zu den Elektrodendurchmessern ist neben dem konkreten Wert des Ausführungsbeispiels zusätzlich jeweils ein bevorzugter Wertebereich in Klammern angegeben.

Die Behandlung mittels Hochfrequenzströmen kann für eine vorgegebene Zeitdauer mit der vorgegebenen Frequenz und Amplitude erfolgen. Ebenso ist es möglich, Programme mit wechselnden Frequenzen in dem Vorgabemittel 2 vorzugeben, welches entsprechend den Generator 1 steuert. Insbesondere sind wechselnde Frequenzen analog zu dem in WO 2009/112181 beschriebenen Frequenzwechsel, vorteilhaft.

Die Vorgabe der Dicke *d* der Dermisschicht durch das Vorgabemittel 2 kann durch zu der Dicke *d* der Dermisschicht korrespondierende Daten erfolgen.

Hierzu gibt der Benutzer lediglich den Behandlungsort am menschlichen Körper vor, beispielsweise Wange, Stirn, Hals oder andere Orte. Hier ist es vorteilhaft, dass das Vorgabemittel eine Auswahlliste mit verschiedenen Behandlungsorten am menschlichen Körper vorgibt, von denen der Benutzer einen Behandlungsort auswählt. In dem Vorgabemittel 2 ist zu dem jeweiligen Behandlungsort jeweils eine typische Dicke der Dermisschicht gespeichert, welche dann für die Bestimmung des optimalen Elektrodenabstands L der bipolaren Elektrode verwendet wird.

Da jedoch bei verschiedenen Menschen die Dicke der Dermisschicht auch an gleichen Behandlungsorten am menschlichen Körper durchaus variiert, ist es zur Erhöhung der Genauigkeit vorteilhaft, dass durch den Benutzer die Dicke der Dermisschicht *d* am Behandlungsort des konkreten, zu behandelnden Menschen vorgegeben wird. Hierzu kann vorteilhafterweise dem Benutzer ein separates Gerät zur Dickenmessung der Dermisschicht bereitgestellt werden.

In einer Variante des zuvor beschriebenen ersten Ausführungsbeispiels ist das Vorgabemittel 2 lediglich als Tabelle, beispielsweise in Papierform vorgegeben. Die Tabelle weist für einen konstanten elektrischen Reflexionskoeffizienten *k* = 0,905 verschiedene Dickenwerte für die Dermisschicht auf und verschiedene Tiefen z, an welchen eine optimale Erwärmung der sWAT-Schicht erfolgen soll. Der Benutzer sucht nun diejenige Zeile in der Tabelle, welche sowohl die nächstliegende Dicke der Dermisschicht, als auch die nächstliegende Tiefe z zu der aktuellen Dicke am Behandlungsort und der gewünschten Behandlungstiefe aufweist. In dieser Zeile ist jeweils diejenige Wechselkappe aufgeführt, welche den für diese Parameterkombination besten Elektrodenabstand *L* der bipolaren Elektrode aufweist. Der Benutzer kann dann die angegebene Wechselkappe auf den Applikator 3 aufstecken und wie zuvor ausgeführt mit der Anwendung beginnen.

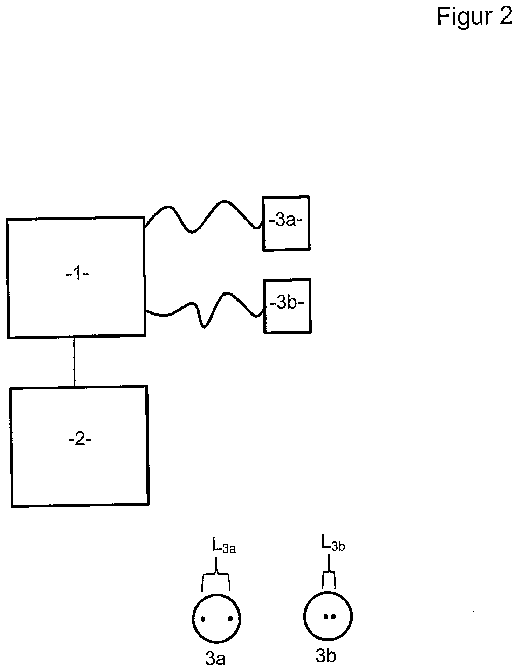

In Figur 2 ist ein zweites Ausführungsbeispiel einer erfindungsgemäßen Vorrichtung gezeigt. Zum Vermeiden von Wiederholungen wird nachfolgend auf die wesentlichen Unterschiede zu dem ersten Ausführungsbeispiel gemäß Figur 1 eingegangen:
Auch die Vorrichtung gemäß Figur 2 weist einen Generator 1 und ein Vorgabemittel 2 auf. Der Generator 1 ist jedoch jeweils über flexible elektrische Leitungen mit einem ersten Applikator 3a und mit einem zweiten Applikator 3b verbunden. In Figur 2 unten ist eine Draufsicht auf die Behandlungsfläche der jeweiligen Applikatoren 3a und 3b gezeigt. Hier ist ersichtlich, dass die bipolare Elektrode des Applikators 3a einen größeren Elektrodenabstand $L_{3a}$ gegenüber der bipolaren Elektrode des Applikators 3b mit Elektrodenabstand $L_{3b}$ aufweist, vorliegend $L_{3a} = 4{,}5$ mm und $L_{3b} = 1$ mm.

Auch bei diesem zweiten Ausführungsbeispiel ermittelt das Vorgabemittel 2 abhängig von der Dicke *d* der Dermisschicht den optimalen Elektrodenabstand *L* und den hierzu nächst liegenden Applikator, welche den dem optimalen Elektrodenabstand nächst kommenden Elektrodenabstand *L* aufweist. Abhängig von diesem Ergebnis wird z.B. ein LED-Licht in dem ausgewählten Applikator angeschaltet, so dass der Benutzer in einfacher Weise anhand dieses optischen Signals den Applikator mit dem geeigneteren Elektrodenabstand *L* für die Anwendung auswählt.

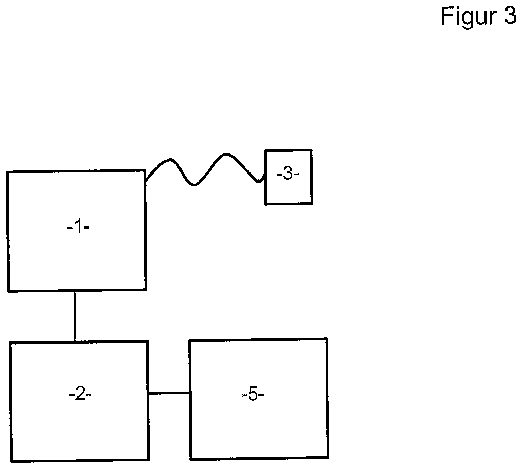

In Figur 3 ist ein drittes Ausführungsbeispiel dargestellt, welches in Grundzügen dem Ausführungsbeispiel gemäß Figur 1 gleicht. Auch hier ist ein Generator 1 und ein Vorgabemittel 2 sowie ein Applikator 3 vorgesehen, wobei der Applikator 3 zur Aufnahme einer der in Figur 1 dargestellten Wechselkappen 4a bis 4d analog zur Beschreibung gemäß Figur 1 ausgebildet ist.

Im Unterschied in der in Figur 1 dargestellten Vorrichtung weist die Vorrichtung gemäß dem in Figur 3 dargestellten Ausführungsbeispiel zusätzlich eine Dickenmesseinheit 5 zur Messung der Dicke einer Dermisschicht auf, welche mit dem Vorgabemittel 2 verbunden ist.

Die Dickenmesseinheit ist als Messeinheit mittels Ultraschall ausgeführt. Der Benutzer verwendet zunächst die Dickenmesseinheit 5, um am Behandlungsort die Dicke der Dermisschicht zu messen. Das Messergebnis wird automatisch an das Vorgabemittel 2 übertragen, welches - wie zu Figur 1 beschrieben - abhängig hiervon den optimalen Elektrodenabstand L und die hierzu nächst liegende Wechselkappe bestimmt.

Zusätzlich zu den bei dem ersten Ausführungsbeispiel beschriebenen Funktionalitäten weist dieses Ausführungsbeispiel noch eine Auswahlmöglichkeit für den Benutzer auf, so dass dieser wahlweise entweder eine Tiefe in der sWAT-Schicht vorgibt, an welcher eine optimale Erwärmung erfolgen soll oder einen Tiefenbereich beginnend mit einer ersten Tiefe *$h_1$* bis zu einer zweiten Tiefe *$h_2$* in der sWAT-Schicht, wobei gilt *$h_1$* kleiner $h_2$. Sofern der Benutzer diesen Modus auswählt, erfolgt eine Ermittlung des optimalen Elektrodenabstands wie vorangehend zu Formel 4 beschrieben.

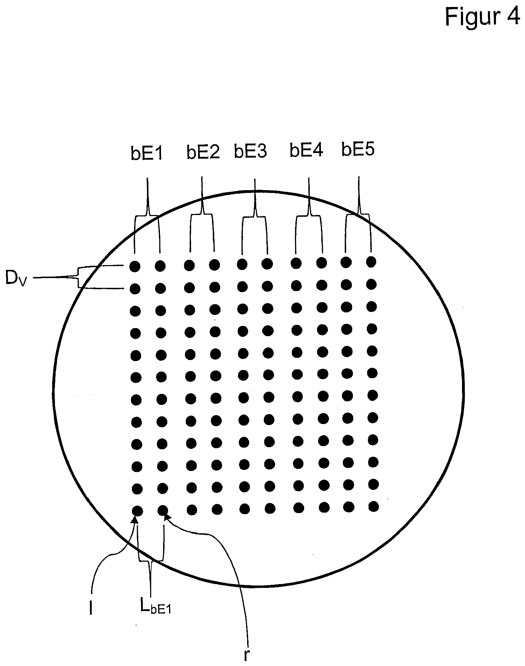

Figur 4 zeigt ein weiteres Ausführungsbeispiel eines Applikators, welcher mehrere bipolare Elektroden umfasst. Darüber hinaus ist jede Elektrode nach Art einer gepunkteten, geraden Linie mit mehreren kreisförmigen Kontaktflächen ausgebildet.

Figur 4 zeigt hierbei die Draufsicht auf die Applikationsfläche des Applikators, welche bei Benutzung auf das Gewebe aufgelegt wird. Der Applikator gemäß des in Figur 4 dargestellten Ausführungsbeispiel weist fünf bipolare Elektroden bE1 bis bE5 auf. Jede dieser fünf bipolaren Elektroden weist jeweils eine nach Art einer gepunkteten, geraden Linie ausgebildete linke und eine gleichartig ausgebildete rechte Elektrode auf. Beispielsweise ist zu der bipolaren Elektrode bE1 die linke Elektrode mit l und die rechte Elektrode mit r gekennzeichnet. Die gepunkteten Linien verlaufen hierbei parallel.

Der Abstand *L* ist somit für alle Kontaktflächen zwischen linker und rechter Elektrode jeweils gleich. Beispielshaft ist für die erste bipolare Elektrode bE1 der Abstand *$L_{bE1}$* gekennzeichnet.

Die Kontaktflächen weisen vorliegend einen Durchmesser von einem Millimeter auf. Der vertikale Abstand *$D_V$* zwischen zwei Kontaktflächen einer Elektrode beträgt zwei Millimeter.

Durch eine Ausbildung gemäß Figur 4 kann somit parallel und gleichzeitig ein Beaufschlagen des Gewebes mittels der dargestellten fünf bipolaren Elektroden erfolgen. Die fünf bipolaren Elektroden sind hierbei jeweils mit dem Generator verbunden.

Die Ausgestaltung gemäß Figur 4 kann bei allen in den Figuren 1 bis 3 dargestellten Ausführungsbeispielen bevorzugt angewendet werden. Bei dem Ausführungsbeispiel gemäß Figur 1 werden in diesem Fall somit die Wechselkappen jeweils gemäß der in Figur 4 dargestellten Draufsicht ausgebildet; bei dem Ausführungsbeispiel gemäß Figur 2 werden die beiden Applikatoren 3a und 3b entsprechend mit mehreren bipolaren Elektroden gemäß der in Figur 4 dargestellten Draufsicht ausgebildet, wobei jeweils sich die Wechselkappen bzw. die Applikatoren hinsichtlich des Abstandes L der Elektroden unterscheiden.

**Patentansprüche**

1. Verfahren zum Einrichten einer Vorrichtung zur Behandlung von Gewebe mittels zumindest einer zumindest bipolaren Elektrode mit zwei in einem Elektrodenabstand L angeordneten Einzelelektroden, wobei der Elektrodenabstand L der Abstand zwischen den Flächenmittelpunkten der Einzelelektroden ist, wobei abhängig

- von der Dicke d einer Dermisschicht des Gewebes an dem zu behandelnden Ort oder hierzu korrespondierender Daten und
- von einem elektrischen Reflexionskoeffizienten k des Gewebes an der der Oberfläche des Gewebes abgewandten Grenze der Dermisschicht an dem zu behandelnden Ort oder hierzu korrespondierender Daten

der Elektrodenabstand L der bipolaren Elektrode vorgegeben wird wobei eine Mehrzahl von Applikatoren zur Auswahl bereitgestellt werden, wobei jeder Applikator zumindest eine bipolare Elektrode aufweist und der Elektrodenabstand L der bipolaren Elektroden der genannten Applikatoren unterschiedlich ist und wobei der Elektrodenabstand L vorgegeben wird, indem ein bestimmter Applikator aus der Mehrzahl von Applikatoren zur Verwendung vorgegeben wird, und dieser Applikator für die Behandlung an dem Behandlungsort ausgewählt wird,

wobei abhängig von der Dicke d der Elektrodenabstand L vorgegeben wird, wobei eine Rechnereinheit vorgesehen ist, in welche die Dicke d durch einen Benutzer eingegeben wird und die Rechnereinheit programmiert ist, den Elektrodenabstand L abhängig von der Dicke d zu vorzugeben
und eine Ultraschall-Dickenmessungseinheit bereitgestellt und die Dicke d des Gewebes am Ort der Anwendung mittels Ultraschallmessung gemessen wird
und eine Auswerteeinheit bereitgestellt wird, um aus den Messdaten der Dickenmessung die Dicke d zu bestimmen, wobei das Ergebnis der Dickenmessung automatisch zur Bestimmung des Elektrodenabstandes L verwendet wird.

2. Verfahren nach Anspruch 1, wobei der bestimmte Applikator durch Vorgabe eines Identifizierungszeichens, insbesondere einer Kennfarbe vorgegeben wird und/oder der bestimmte Applikator durch ein optisches Signal, insbesondere ein Leuchtsignal an diesem Applikator vorgegeben wird.

3. Verfahren nach Anspruch 1, wobei ein Applikator mit einer bipolaren Elektrodenvorrichtung bereitgestellt wird, bei welchem mittels einer Steuereinheit unterschiedliche Elektrodenabstände L zwischen den Elektroden einstellbar sind und wobei der Elektrodenabstand L vorgegeben wird, indem die Steuereinheit auf den Elektrodenabstand L konfiguriert wird.

4. Vorrichtung zur Behandlung von menschlichem Gewebe mittels Hochfrequenzströmen, mit einem Generator zum Erzeugen von Hochfrequenzströmen, und zumindest einer ersten und einer zweiten bipolaren Elektrode, welche jeweils zwei in einem Elektrodenabstand $L$ angeordneten Einzelelektroden aufweisen, wobei der Elektrodenabstand $L$ der Abstand zwischen den Flächenmittelpunkten der Einzelelektroden ist und welche bipolaren Elektroden mit dem Generator elektrisch leitend verbindbar sind, wobei die beiden bipolaren Elektroden einen unterschiedlichen Elektrodenabstand L zwischen den Einzelelektroden aufweisen, wobei ein Vorgabemittel zur Vorgabe des Elektrodenabstands L abhängig

- von der Dicke d einer Dermisschicht des Gewebes an dem zu behandelnden Ort oder hierzu korrespondierender Daten und
- von einem elektrischen Reflexionskoeffizienten kos des Gewebes an der der Oberfläche des Gewebes abgewandten Grenze der Dermisschicht an dem zu behandelnden Ort oder hierzu korrespondierender Daten

vorgesehen ist und das Vorgabemittel eine Eingabeeinheit zur Eingabe

- der von der Dicke d der Dermisschicht an dem zu behandelnden Ort oder hierzu korrespondierender Daten und/oder
- von einem elektrischen Reflexionskoeffizienten k des Gewebes an der der Oberfläche des Gewebes abgewandten Grenze der Dermisschicht an dem zu behandelnden Ort oder hierzu korrespondierender Daten und eine Auswerteeinheit zur Bestimmung des vorzugebenden Elektrodenabstandes L aufweist,

und die Vorrichtung eine Ultraschall-Dickenmesseinheit zur Dickenmessung der Dicke d der Dermisschicht umfasst und die Ultraschall-Dickenmesseinheit mit dem Vorgabemittel zusammenwirkend ausgebildet ist, um eine gemessene Dicke d an das Vorgabemittel automatisch weiterzuleiten.

5. Vorrichtung nach Anspruch 4, wobei die Vorrichtung einen Applikator, welcher elektrisch leitend mit dem Generator verbunden ist und zumindest eine erste Wechselkappe mit der ersten bipolaren Elektrode und eine zweite Wechselkappe mit der zweiten bipolaren Elektrode aufweist und die Wechselkappen wahlweise an dem Applikator anordbar ausgebildet sind, derart, dass die bipolare Elektrode der an dem Applikator angeordneten Wechselkappe elektrisch leitend mit dem Generator verbunden ist, wobei bevorzugt, die Wechselkappen eine unterschiedliche Kennung, bevorzugt eine farbliche Kennung aufweisen, insbesondere, die Vorrichtung eine Kennungsanzeigeeinheit umfasst, welche ausgebildet ist, abhängig von dem vorgegebenen Elektrodenabstand L die Kennung der entsprechenden Wechselkappe auszugeben.

**6.** Vorrichtung nach Anspruch 4, wobei die Vorrichtung zumindest einen ersten und einen zweiten Applikator aufweist, wobei die erste Elektrode an dem ersten Applikator und die zweite Elektrode an dem zweiten Applikator angeordnet ist, wobei jeder Applikator eine optische Anzeige, insbesondere ein Leuchtmittel aufweist und wobei das Vorgabemittel elektrisch mit der optischen Anzeige jedes Applikators verbunden und derart ausgebildet ist, dass abhängig von dem vorgegebenen Elektrodenabstand $L$ die optische Anzeige des entsprechenden Applikators aktiviert wird.

**Claims**

**1.** Method for setting up a device for treating tissue by means of at least one at least bipolar electrode having two individual electrodes arranged at an electrode spacing $L$, wherein the electrode spacing $L$ is the spacing between the centroids of the individual electrodes, wherein
the electrode spacing $L$ of the bipolar electrode is specified in dependence

- upon the thickness $d$ of a dermal layer of the tissue at the location to be treated or data corresponding thereto and
- upon an electrical reflection coefficient $k$ of the tissue at the boundary of the dermal layer remote from the surface of the tissue at the location to be treated or data corresponding thereto,

wherein a plurality of applicators are provided for selection, each applicator having at least one bipolar electrode and the electrode spacing $L$ of the bipolar electrodes of the said applicators being different, and

wherein the electrode spacing $L$ is specified by specifying a particular applicator from the plurality of applicators for use, and that applicator is selected for the treatment at the treatment location,
wherein the electrode spacing $L$ is specified in dependence upon the thickness $d$, a computer unit being provided into which the thickness d is input by a user and the computer unit being programmed to specify the electrode spacing $L$ in dependence upon the thickness $d$,
and an ultrasonic thickness measurement unit is provided and the thickness $d$ of the tissue at the location of use is measured by means of ultrasonic measurement,
and an analysis unit is provided in order to determine the thickness d from the measurement data of the thickness measurement, the result of the thickness measurement being used automatically for determining the electrode spacing $L$.

**2.** Method according to claim 1,
wherein the particular applicator is specified by specifying an identification symbol, especially a characteristic colour, and/or the particular applicator is specified by an optical signal, especially a light signal on that applicator.

**3.** Method according to claim 1,

wherein an applicator having a bipolar electrode device is provided, for which different electrode spacings $L$ between the electrodes can be set by means of a control unit, and
wherein the electrode spacing $L$ is specified by configuring the control unit to the electrode spacing $L$.

**4.** Device for treating human tissue by means of high frequency currents, having a generator for generating high frequency currents, and at least one first and one second bipolar electrode which each have two individual electrodes arranged at an electrode spacing $L$,
wherein the electrode spacing $L$ is the spacing between the centroids of the individual electrodes and which bipolar electrodes are electrically conductively connectible to the generator, the two bipolar electrodes having a different electrode spacing $L$ between the individual electrodes, a specifying means being provided for specifying the electrode spacing $L$ in dependence

- upon the thickness $d$ of a dermal layer of the tissue at the location to be treated or data corresponding thereto and
- upon an electrical reflection coefficient $k_{DS}$ of the tissue at the boundary of the dermal layer remote from the surface of the tissue at the location to be treated or data corresponding thereto,

and
the specifying means has an input unit for inputting

- the thickness *d* of a dermal layer at the location to be treated or data corresponding thereto, and/or
- an electrical reflection coefficient *k of* the tissue at the boundary of the dermal layer remote from the surface of the tissue at the location to be treated or data corresponding thereto,

and an analysis unit for determining the electrode spacing *L* to be specified,
and the device comprises an ultrasonic thickness measurement unit for measuring the thickness *d* of the dermal layer, and the ultrasonic thickness measurement unit is designed to co-operate with the specifying means in order automatically to forward a measured thickness *d* to the specifying means.

5. Device according to claim 4,
wherein the device has an applicator, which is in electrically conductive connection with the generator, and at least one first interchangeable cap having the first bipolar electrode and a second interchangeable cap having the second bipolar electrode, and the interchangeable caps are designed to be mounted selectively on the applicator in such a way that the bipolar electrode of the interchangeable cap arranged on the applicator is in electrically conductive connection with the generator, wherein preferably the interchangeable caps have different identifiers, preferably a colour identifier; especially, the device comprises an identifier display unit which is designed to output the identifier of the corresponding interchangeable cap in dependence upon the specified electrode spacing *L.*

6. Device according to claim 4,
wherein the device has at least one first and one second applicator, the first electrode being arranged on the first applicator and the second electrode being arranged on the second applicator, each applicator having an optical display, especially a light means, and wherein the specifying means is electrically connected to the optical display of each applicator and is designed in such a way that the optical display of the corresponding applicator is activated in dependence upon the specified electrode spacing *L.*

**Revendications**

1. Procédé pour créer un dispositif pour le traitement d'un tissu au moyen d'au moins une électrode au moins bipolaire avec deux électrodes individuelles disposées dans un écartement d'électrodes L, dans lequel l'écartement d'électrodes L est la distance entre les centres de surface des électrodes individuelles,

dans lequel
l'écartement d'électrodes L de l'électrode bipolaire est prédéfini en fonction

- de l'épaisseur d d'une couche de derme du tissu sur le lieu à traiter ou de données correspondant à celle-ci

et

- d'un coefficient de réflexion électrique k du tissu sur la limite de la couche de derme opposée à la surface du tissu sur le lieu à traiter ou de données correspondant à celui-ci

dans lequel une pluralité d'applicateurs à choisir sont fournis, dans lequel chaque applicateur présente au moins une électrode bipolaire et l'écartement d'électrodes L des électrodes bipolaires des applicateurs cités est différent et
dans lequel
l'écartement d'électrodes L est prédéfini du fait qu'un applicateur donné à partir de la pluralité d'applicateurs à utiliser est prédéfini, et cet applicateur est sélectionné pour le traitement au lieu de traitement,
dans lequel l'écartement d'électrodes L est prédéfini en fonction de l'épaisseur d, dans lequel une unité de calcul est prévue, dans laquelle l'épaisseur d est entrée par un utilisateur et l'unité de calcul est programmée pour prédéfinir l'écartement d'électrodes L en fonction de l'épaisseur d
et une unité de mesure d'épaisseur à ultrasons est fournie et l'épaisseur d du tissu est mesurée sur le lieu de l'utilisation au moyen d'une mesure à ultrasons
et une unité d'évaluation est fournie, afin de déterminer à partir des données de mesure de la mesure d'épaisseur l'épaisseur d, dans lequel le résultat de la mesure d'épaisseur est utilisé automatiquement pour la détermination de l'écartement d'électrodes L.

2. Procédé selon la revendication 1,

dans lequel
l'applicateur donné est prédéfini par prédéfinition d'un signe d'identification, en particulier d'une couleur distinctive et/ou
l'applicateur donné est prédéfini par un signal optique, en particulier un signal lumineux sur cet applicateur.

3. Procédé selon la revendication 1,
dans lequel un applicateur avec un dispositif d'électrode bipolaire est fourni, pour lequel des écartements d'électrodes L différents entre les électrodes peuvent être réglés au moyen d'une unité de commande et dans lequel l'écartement d'électrodes L est prédéfini, du fait que l'unité de commande est configurée sur l'écartement d'électrodes L.

4. Dispositif pour le traitement d'un tissu humain au moyen de courants de haute fréquence,

avec un générateur pour générer des courants de haute fréquence, et au moins une première et une deuxième électrode bipolaire, lesquelles présentent respectivement deux électrodes individuelles disposées dans un écartement d'électrodes L, dans lequel l'écartement d'électrodes L est l'espacement entre les points de surface des électrodes individuelles et lesquelles électrodes bipolaires peuvent être connectées de manière électro-conductrice au générateur, dans lequel les deux électrodes bipolaires présentent un écartement d'électrodes L différent entre les électrodes individuelles,
dans lequel un moyen de prédéfinition est prévu pour la prédéfinition de l'écartement d'électrodes L en fonction

- de l'épaisseur d d'une couche de derme du tissu sur le lieu à traiter ou de données correspondant à celle-ci

et

- d'un coefficient de réflexion électrique $k_{DS}$ du tissu sur la limite de la couche de derme opposée à la surface du tissu sur le lieu à traiter ou de données correspondant à celui-ci

et
le moyen de prédéfinition présente une unité d'entrée pour l'entrée

- de de l'épaisseur d de la couche de derme sur le lieu à traiter ou des données correspondant à celle-ci et/ou
- d'un coefficient de réflexion électrique k du tissu sur la limite de la couche de derme opposée à la surface du tissu sur le lieu à traiter ou de données correspondant à celui-ci et une unité d'évaluation pour la détermination de l'écartement d'électrodes L à prédéfinir,

et le dispositif comprend une unité de mesure d'épaisseur à ultrasons pour la mesure d'épaisseur de l'épaisseur d de la couche de derme et l'unité de mesure d'épaisseur à ultrasons est réalisée de manière à coopérer avec le moyen de prédéfinition, afin de transmettre automatiquement une épaisseur d mesurée au moyen de prédéfinition.

5. Dispositif selon la revendication 4,

dans lequel le dispositif présente un applicateur, lequel est connecté au générateur de manière électro-conductrice et présente au moins un premier capuchon interchangeable avec la première électrode bipolaire et un deuxième capuchon interchangeable avec la deuxième électrode bipolaire et les capuchons interchangeables sont réalisés de manière à pouvoir être disposés sélectivement sur l'applicateur, de telle sorte que l'électrode bipolaire du capuchon interchangeable disposé sur l'applicateur soit reliée au générateur de manière électro-conductrice,
dans lequel de préférence,
les capuchons interchangeables présentent un code différent, de préférence un code couleur, en particulier, le dispositif comprend une unité d'affichage de code, laquelle est réalisée pour délivrer le code du capuchon interchangeable correspondant en fonction de l'écartement d'électrodes L prédéfini.

6. Dispositif selon la revendication 4,

dans lequel
le dispositif présente au moins un premier et un deuxième applicateur, dans lequel la première électrode est disposée sur le premier applicateur et la deuxième électrode sur le deuxième applicateur, dans lequel chaque applicateur présente un affichage optique, en particulier un moyen d'éclairage et dans lequel le moyen de prédéfinition est connecté électriquement à l'affichage optique de chaque applicateur et réalisé de telle sorte que l'affichage optique de l'applicateur correspondant soit activé en fonction de l'écartement d'électrodes L prédéfini.

Figur 1

Figur 2

Figur 3

Figur 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10224154 A1
- DE 69733556 T2
- EP 0873722 A1
- DE 60312348 T2
- US 20100211060 A1
- US 2008183251 A1
- WO 2009112181 A

**In der Beschreibung aufgeführte Nicht-Patentliteratur**

- **KRACKOWIZER, P. ; BRENNER, E.** *Dicke der Epidermis und Dermis, Phlebologie,* 2008, vol. 8, 83-92